# EUROPEAN PATENT APPLICATION

(11) **EP 4 289 955 A1**
(43) Date of publication of application: **13.12.2023**
(21) Application number: 22749862.3
(22) Date of filing: 08.02.2022
(51) Int. Cl.: C12N 15/31, C07K 14/245, C12N 1/21, C12P 19/00, A61K 31/702, A61P 1/12, A61P 25/28, A61P 37/04

(54) **PROTEIN HAVING FUCOSE-CONTAINING SUGAR TRANSPORTING ACTIVITY, AND METHOD FOR PRODUCING FUCOSE-CONTAINING SUGAR**

(30) Priority: 08.02.2021 JP 2021018484
(71) Applicant: Kyowa Hakko Bio Co., Ltd., Tokyo 100-8185 (JP)
(72) Inventor: SUGITA Tomotoshi, Tokyo 100-8185 (JP)
(74) Representative: Vossius & Partner Patentanwälte Rechtsanwälte mbB
(86) International application number: PCT/JP2022/004972
(87) International publication number: WO 2022/168991

(57) **Abstract**

An object of the present invention is to provide a method for efficiently producing a fucose-containing carbohydrate by using a protein associated with transport of a fucose-containing carbohydrate and a microorganism having an ability to produce the protein. The present invention relates to any one protein of [1] a protein consisting of the amino acid sequence represented by SEQ ID NO: 2 or 4, [2] a mutant protein having a transporting activity for a fucose-containing carbohydrate and consisting of an amino acid sequence in which 1 to 20 amino acids are deleted, substituted, inserted, or added in the amino acid sequence represented by SEQ ID NO: 2 or 4, and [3] a homologous protein having a transporting activity for a fucose-containing carbohydrate and consisting of an amino acid sequence having an identity of 90% or more with the amino acid sequence represented by SEQ ID NO: 2 or 4.

## Description

### Technical Field

The present invention relates to a transporter associated with excretion of a fucose-containing carbohydrate and a method for producing a fucose-containing carbohydrate such as 3-fucosyllactose.

### Background Art

It has been reported that human milk oligosaccharides (HMO) contained in human milk have health functions such as improving the intestinal environment as a prebiotic, activating immunity, and developing cognitive functions in infants, and from a physiological activity thereof, they are expected to be used as an additive for infant formula and as a health functional ingredient for adults (Non Patent Literature 1).

Among carbohydrates known as HMOs, fucose-containing carbohydrates such as 2'-fucosyllactose and 3-fucosyllactose account for about 60% of the total HMOs in terms of substance amount ratio (Non Patent Literature 2), and among HMOs, particular attention is paid to functionality thereof.

As a method for producing a fucose-containing carbohydrate, a microbial fermentation method using a fucosyltransferase is widely used. Non Patent Literature 3 discloses a method for producing 2'-fucosyllactose or 3-fucosyllactose by a fermentation method using a microorganism expressing a fucosyltransferase derived from a microorganism such as Helicobacter pylori or Bacteroides fragilis and using lactose and GDP-fucose as substrates.

Patent Literature 1 shows that a SetA protein known as a transporter associated with excretion of a saccharide in Escherichia coli is also associated with transport of a fucose-containing carbohydrate such as 2'-fucosyllactose, and discloses a method for improving fucose-containing carbohydrate productivity by overexpressing the protein.

The SetA protein is a transport protein belonging to the SET family among the major facilitator superfamily (MFS) transporters (Non Patent Literature 4), but no other proteins belonging to the same SET family and associated with the transport of a fucose-containing carbohydrate are known.

It is disclosed that a CDT2 protein derived from Neurospora crassa belonging to the SP family, as a transporter in charge of transport of oligosaccharides in the same manner as the SetA protein, is associated with transport of 2'-fucosyllactose (Non Patent Literature 5).

### Citation List

### Patent Literature

Patent Literature 1: Japanese Patent No. 5580408

### Non Patent Literature

Non Patent Literature 1: Int J Pediatrics (2019) 2390240: 1-8
Non Patent Literature 2: Curr Opin Biotechnol (2019) 56: 130-137
Non Patent Literature 3: Metabolic Engineering (2017) 41: 23-38
Non Patent Literature 4: J Biol Chem (1999) 274: 22977-22984
Non Patent Literature 5: Metabolic Engineering (2019) 52: 232-242

### Summary of Invention

### Technical Problem

However, other than the SetA protein and the CDT2 protein, a protein associated with excretion of a fucose-containing carbohydrate has not been reported, and further search for a protein associated with transport of a fucose-containing carbohydrate is required as a method for implementing a method for efficiently producing a fucose-containing carbohydrate.

Accordingly, an object of the present invention is to provide a method for efficiently producing a fucose-containing carbohydrate by using a protein associated with transport of a fucose-containing carbohydrate and a microorganism having an ability to produce the protein.

### Solution to Problem

The present inventor has found that 3-fucosyllactose can be efficiently produced by using a microorganism having an ability to produce a protein having a transporting activity for a fucose-containing carbohydrate and comprising a specific amino acid sequence, as compared with a method in related art, and has completed the present invention.

That is, the present invention is as follows.
1. A protein of any one of [1] to [3] below,
   [1] a protein consisting of the amino acid sequence represented by SEQ ID NO: 2 or 4,
   [2] a mutant protein having a transporting activity for a fucose-containing carbohydrate and consisting of an amino acid sequence in which 1 to 20 amino acids are deleted, substituted, inserted, or added in the amino acid sequence represented by SEQ ID NO: 2 or 4, and
   [3] a homologous protein having a transporting activity for a fucose-containing carbohydrate and consisting of an amino acid sequence having an identity of 90% or more with the amino acid sequence represented by SEQ ID NO: 2 or 4.
2. The protein according to the above 1, in which the fucose-containing carbohydrate is an oligosaccharide.
3. A DNA consisting of the nucleotide sequence represented by SEQ ID NO: 1 or 3 or a homologous sequence thereof and encoding the protein according to the above 1 or 2.
4. A recombinant DNA comprising the DNA according to the above 3.
5. A transformant obtained by transforming a host cell with the recombinant DNA according to the above 4.
6. The transformant according to the above 5, which is a microorganism with an enhanced activity of the protein of any one of [1] to [3] according to the above 1 and enhanced productivity of the fucose-containing carbohydrate.
7. The transformant according to the above 6, in which the microorganism is Escherichia coli having an ability to produce the fucose-containing carbohydrate.
8. A method for producing a fucose-containing carbohydrate, including: culturing the transformant according to any one of the above 5 to 7 in a culture medium to produce the fucose-containing carbohydrate in a culture product.
9. The production method according to the above 8, in which the fucose-containing carbohydrate is an oligosaccharide.
10. The production method according to the above 9, in which the oligosaccharide is 3-fucosyllactose.

### Advantageous Effects of Invention

The protein of the present invention has an excellent transporting activity for a fucose-containing carbohydrate due to a specific amino acid sequence thereof. By using a microorganism having an ability to produce the protein of the present invention, a fucose-containing carbohydrate such as 3-fucosyllactose can be efficiently produced as compared with that in related art.

### Description of Embodiments

### 1. Protein of Present Invention

A protein of the present invention is a protein according to any one of [1] to [3] below.
[1] a protein consisting of the amino acid sequence represented by SEQ ID NO: 2 or 4,
[2] A mutant protein having a transporting activity for a fucose-containing carbohydrate and consisting of an amino acid sequence in which 1 to 20 amino acids are deleted, substituted, inserted, or added in the amino acid sequence represented by SEQ ID NO: 2 or 4.
[3] A homologous protein having a transporting activity for a fucose-containing carbohydrate and consisting of an amino acid sequence having an identity of 90% or more with the amino acid sequence represented by SEQ ID NO: 2 or 4.

The expression "deletion of an amino acid" means deletion or elimination of an amino acid residue in a sequence, the expression "substitution of an amino acid" means substitution of an amino acid residue in a sequence with another amino acid residue, the expression "insertion of an amino acid" means insertion of a new amino acid in a sequence, and the expression "addition of an amino acid" means addition of a new amino acid residue so as to be inserted into a sequence.

As a specific embodiment of the "deletion, substitution, insertion, or addition of 1 to 20 amino acids", there is an embodiment in which 1 to 20 amino acids are substituted with other chemically similar amino acids. Examples thereof include a case where a certain hydrophobic amino acid is substituted with another hydrophobic amino acid, and a case where a certain polar amino acid is substituted with another polar amino acid having the same charge. Such chemically similar amino acids are known in the art for each amino acid.

Specific examples of the non-polar (hydrophobic) amino acid include alanine, valine, glycine, isoleucine, leucine, proline, tryptophan, phenylalanine, and methionine. Examples of the polar (neutral) amino acid include serine, threonine, tyrosine, glutamine, asparagine, and cysteine. Examples of the positively charged basic amino acid include arginine, histidine, and lysine. Examples of the negatively charged acidic amino acid include aspartic acid and glutamic acid.

Examples of the amino acid sequence in which 1 to 20 amino acids are deleted, substituted, inserted, or added in an amino acid sequence of a target protein include an amino acid sequence having a sequence identity of a certain degree or more with the amino acid sequence of the target protein. Examples thereof include an amino acid sequence having an identity of preferably 60% or more, more preferably 65% or more, even more preferably 70% or more, still more preferably 75% or more, even still more preferably 80% or more, yet still more preferably 85% or more, further even still more preferably 90% or more, and particularly preferably 95% or more with the amino acid sequence of the target protein.

The fact that 1 to 20 amino acids are deleted, substituted, inserted, or added in the amino acid sequence represented by SEQ ID NO: 2 or 4 can be confirmed by, for example, aligning an amino acid sequence of a protein to be confirmed as having the deletion, substitution, insertion, or addition with an amino acid sequence of an original protein.

The alignment of the amino acid sequences can be created by using, for example, a well-known alignment program ClustalW [Nucelic Acids Research 22, 4673, (1994)]. ClustalW is available, for example, from http://www. ebi.ac. uk/clustalw/ (European Bioinformatics Institute). For example, a default value can be used for parameters when creating an alignment using ClustalW.

When 1 to 20 amino acids are substituted, inserted, or added in the amino acid sequence represented by SEQ ID NO: 2 or 4, an amino acid residue to be substituted, inserted, or added may be natural or non-natural. Examples of the natural amino acid include L-alanine, L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, o-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

The transporting activity for a fucose-containing carbohydrate refers to an activity of transporting an intracellular fucose-containing carbohydrate to the outside of the cell.

For example, the fucose-containing carbohydrate is preferably an oligosaccharide containing fucose as a constitutive monosaccharide, and more preferably an oligosaccharide having a chain length of 3 to 6 and having a lactose end. More specific examples thereof include 3-fucosyllactose, 2'-fucosyllactose, lacto-N-fucopentaose I, lacto-N-fucopentaose II, lacto-N-fucopentaose III, lacto-N-fucopentaose V, lactodifucotetraose, lacto-N-difcohexaose I, and lacto-N-difcohexaose II. Among them, 3-fucosyllactose is exemplified.

The mutant protein refers to a protein obtained by artificially deleting or substituting an amino acid residue of an original protein or artificially inserting or adding an amino acid residue in the protein. The expression "an amino acid is deleted, substituted, inserted, or added in the mutant protein may mean that 1 to 20 amino acids are deleted, substituted, inserted, or added at any position in the same sequence.

An amino acid to be substituted, inserted, or added may be natural or non-natural. Examples of the natural amino acid include the above-described natural amino acids. Examples of mutually substitutable amino acids are as described above. Amino acids contained in the same group can be mutually substituted.

The homologous protein refers to a group of proteins derived from proteins of the same evolutionary origin, which are proteins possessed by organisms existing in nature. The homologous proteins are similar in structure and function to each other.

The identity of amino acid sequences and nucleotide sequences can be determined using an algorithm BLAST (Pro. Nat. Acad. Sci. USA, 90, 5873, 1993) or FASTA (Methods Enzymol., 183, 63, 1990) according to Karlin and Altschul. Based on the algorithm BLAST, a program called BLASTN or BLASTX has been developed (J. Mol. Biol., 215, 403, 1990). When analyzing a nucleotide sequence using BLASTN based on BLAST, parameters are, for example, Score = 100 and wordlength = 12. When analyzing an amino acid sequence using BLASTX based on BLAST, parameters are, for example, score = 50 and wordlength = 3. When using BLAST and Gap ped BLAST programs, use default parameters for each program. A specific method of the analysis method is known.

It can be confirmed, for example, by the following method that the above mutant protein or homologous protein has a transporting activity for a fucose-containing carbohydrate. First, a recombinant DNA comprising a DNA encoding the above mutant protein or homologous protein whose activity is to be confirmed is prepared by a method described later. Next, a transformant having a higher activity of the protein than a parent strain is prepared by transforming the parent strain with the recombinant DNA, and amounts of fucose-containing carbohydrates produced and accumulated in culture solutions of the parent strain and the transformant are compared to confirm. In the present description, the term "parent strain" refers to an original strain to be subjected to genetic modification, transformation, and the like.

### 2. DNA of Present Invention

A DNA of the present invention is a DNA encoding the protein according to the above [1] to [3]. Specific examples of the DNA of the present invention include DNAs of [A1] to [A3] below.
[A1] The nucleotide sequence represented by SEQ ID NO: 1 or 3.
[A2] A DNA hybridizing with a DNA consisting of a nucleotide sequence complementary to the nucleotide sequence represented by SEQ ID NO: 1 or 3 under stringent conditions and encoding a homologous protein having a transporting activity for a fucose-containing carbohydrate.
[A3] A DNA consisting of a nucleotide sequence having an identity of 95% or more, preferably 97% or more, more preferably 98% or more, and most preferably 99% or more with the nucleotide sequence represented by SEQ ID NO: 1 or 3 and encoding a homologous protein having a transporting activity for a fucose-containing carbohydrate.

In the above description, the term "hybridizing" refers to a step in which a DNA hybridizes to a DNA comprising a specific nucleotide sequence or a part of the DNA. Accordingly, a nucleotide sequence of the DNA hybridizing to a DNA comprising a specific nucleotide sequence or a part of the DNA may be useful as a probe for Northern or Southern blot analysis, or may be a DNA having a length that can be used as an oligonucleotide primer for PCR analysis.

Examples of the DNA used as a probe include DNAs having at least 100 bases or more, preferably 200 bases or more, and more preferably 500 bases or more. Examples of the DNA used as a primer include DNAs having at least 10 bases or more, and preferably 15 bases or more.

A method for a DNA hybridization experiment is well known, and for example, Molecular Cloning, 4th Edition (Cold Spring Harbor Laboratory Press, 2012), Methods for General and Molecular Bacteriology (ASM Press, 1994), Immunology methods manual (Academic press, 1997), and many other standard textbooks can be followed to determine hybridization conditions and perform experiments.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mM sodium chloride, 75 mM sodium citrate), 50 mM sodium phosphate (pH 7.6), a 5× Denhardt's solution, 10% dextran sulfate, and a 20 µg/L of denatured salmon sperm DNA, and then the filter is washed in a 0.2×SSC solution at, for example, about 65°C.

The above various conditions can also be set by adding or changing a blocking reagent used to reduce background in a hybridization experiment. The addition of the above blocking reagent may be accompanied by a change in hybridization conditions in order to meet the conditions.

Examples of the DNA capable of hybridizing under the above stringent conditions include a DNA consisting of a nucleotide sequence having an identity of 95% or more, preferably 97% or more, more preferably 98% or more, and most preferably 99% or more with the nucleotide sequence represented by SEQ ID NO: 1 or 3 when calculated based on the above parameters using the above program such as BLAST or FASTA.

The DNA of the present invention can be obtained by, for example, using a DNA encoding a protein consisting of the amino acid sequence represented by SEQ ID NO: 2 or 4, introducing a mutation by a site-directed mutagenesis method described in, for example, Molecular Cloning 4th Edition (Cold Spring Harbor Laboratory Press, 2012) and Current Protocols in Molecular Biology (JOHN WILEY & SONS, INC.), and substituting with a nucleotide sequence encoding another amino acid residue. Alternatively, the DNA of the present invention can also be obtained by using a PrimeSTAR Mutagenesis Basal Kit (manufactured by Takara Bio Inc.), or the like.

The DNA encoding the protein consisting of the amino acid sequence represented by SEQ ID NO: 2 can be obtained by, for example, Southern hybridization to a chromosomal DNA library of a microorganism, preferably of the genus Escherichia microorganism, and more preferably of an Escherichia coli W3110 strain using a probe that can be designed based on the nucleotide sequence of the DNA encoding the protein consisting of the amino acid sequence represented by SEQ ID NO: 2, or by PCR [PCR Protocols, Academic press (1990)] using a primer DNA that can be designed based on the DNA encoding the protein comprising the amino acid sequence represented by SEQ ID NO: 2 and using, as a template, a chromosomal DNA of the Escherichia coli W3110 strain. Specific examples of the DNA encoding the protein consisting of the amino acid sequence represented by SEQ ID NO: 2 include a DNA consisting of the nucleotide sequence represented by SEQ ID NO: 1.

The DNA encoding the amino acid sequence represented by SEQ ID NO: 4 can be obtained by, for example, Southern hybridization to a chromosomal DNA library of a microorganism, preferably of the genus Escherichia microorganism, and more preferably of an Escherichia coli W3110 strain using a probe that can be designed based on the nucleotide sequence of the DNA encoding the protein consisting of the amino acid sequence represented by SEQ ID NO: 4, or by PCR using a primer DNA that can be designed based on the DNA encoding the protein consisting of the amino acid sequence represented by SEQ ID NO: 4 and using, as a template, a chromosomal DNA of the Escherichia coli W3110 strain. Specific examples of the DNA encoding the protein consisting of the amino acid sequence represented by SEQ ID NO: 4 include a DNA consisting of the nucleotide sequence represented by SEQ ID NO: 3.

The DNA encoding the mutant protein having a transporting activity for a fucose-containing carbohydrate and comprising an amino acid sequence in which 1 to 20 amino acids are deleted, substituted, inserted, or added in the amino acid sequence represented by SEQ ID NO: 2 or 4 according to the above [2] can be obtained by, for example, subjecting the DNA consisting of the nucleotide sequence represented by SEQ ID NO: 1 or 3 to error-prone PCR or the like as a template.

Alternatively, the DNA encoding the mutant protein having a transporting activity for a fucose-containing carbohydrate and comprising an amino acid sequence in which 1 to 20 amino acids are are deleted, substituted, inserted, or added in the amino acid sequence represented by SEQ ID NO: 2 or 4 according to the above [2] can also be obtained by a partially directed mutagenesis method by PCR using a set of PCR primers each having a nucleotide sequence designed to introduce a desired mutation (deletion, substitution, insertion, or addition) at the 5' end (Gene, 77, 51, 1989).

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced is obtained.

The DNA encoding the homologous protein having a transporting activity for a fucose-containing carbohydrate and comprising an amino acid sequence having an identity of 90% or more with the amino acid sequence represented by SEQ ID NO: 2 or 4 can be obtained by, for example, the following method. Specifically, for example, the DNA encoding the homologous protein can be obtained by a method similar to the method for obtaining the DNA encoding the protein comprising the amino acid sequence represented by SEQ ID NO:2 or 4, using a probe DNA or primer DNA that can be designed based on a nucleotide sequence or an amino acid sequence obtained by searching various gene sequence databases for a nucleotide sequence having an identity of preferably 80% or more, more preferably 90% or more, even more preferably 95% or more, and most preferably 99% or more with the nucleotide sequence represented by SEQ ID NO: 1 or 3, and a microorganism having the DNA.

The identity of the nucleotide sequence and the amino acid sequence can be determined by a method same as that described in the above 1. A nucleotide sequence of the DNA of the present invention obtained by the above method can be determined by using the DNA as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method (Proc. Nat. Acad. Sci., USA, 74, 5463, 1977) or a nucleotide sequence analyzer such as an Applied Biosystems 3500 Genetic Analyzer and an Applied Biosystems 3730 DNA Analyzer (both manufactured by Thermo Fisher Scientific K.K.).

Examples of the vector that can be used for determining the nucleotide sequence of the DNA of the present invention include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990).

The above host cell may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

As a method for introducing the recombinant DNA obtained by incorporating the DNA of the present invention into a host cell, any method for introducing a DNA into a host cell can be used, and examples thereof include a method using calcium ions (Proc. Natl. Acad. Sci., USA, 69, 2110, 1972), a protoplast method (JPS63-248394A), and an electroporation method (Nucleic Acids Res., 16, 6127, 1988).

When the obtained DNA is partial length as a result of determining the nucleotide sequence, the full-length DNA can be obtained by a Southern hybridization method or the like for a chromosomal DNA library using the partial length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

### 3. Recombinant DNA of Present Invention

A recombinant DNA of the present invention is a DNA autonomously replicatable in a host cell, and is obtained by incorporating the DNA of the present invention of the above 2 into an expression vector comprising a promoter at a position where the DNA of the present invention can be transcribed.

A DNA capable of being incorporated into a chromosome in a host cell and comprising the DNA of the present invention is also the recombinant DNA of the present invention. When the recombinant DNA is a recombinant DNA capable of being incorporated into a chromosome, the recombinant DNA may not contain a promoter.

When a prokaryote such as bacteria is used as a host cell, the recombinant DNA of the present invention is preferably a recombinant DNA composed of a promoter, a ribosomal binding sequence, the DNA of the present invention of the above 2, and a transcription termination sequence. Further, a gene controlling a promoter may be comprised.

Here, it is preferred to adjust a distance between a shine-dalgarno sequence, which is a ribosomal binding sequence, and an initiation codon to an appropriate distance, for example, 6 to 18 bases. In the recombinant DNA of the present invention, the transcription termination sequence is not necessarily required for the expression of the DNA of the present invention, but it is preferred to place a transcription termination sequence immediately below the structural gene.

When a microorganism belonging to the genus Escherichia is used as a host cell into which the recombinant DNA of the present invention is to be introduced, examples of the expression vector include pColdI, pSTV28, and pUC118 (all manufactured by Takara Bio Inc.), pET21a, pCDF-1b, and pRSF-1b (all manufactured by Merck Millipore Inc.), pMAL-c5x (manufactured by New England Biolabs), pGEX-4T-1 and pTrc99A (both manufactured by GE Healthcare Bioscience), pTrcHis and pSE280 (both manufactured by Thermo Fisher Scientific K.K.), pGEMEX-1 (manufactured by Promega Corporation), pQE-30, pQE-60, and pQE80L (all manufactured by Qiagen), pET-3, pBluescriptII SK(+), and pBluescriptII KS(-) (all manufactured by Agilent Technologies, Inc.), pKYP10 (JPS58-110600A), pKYP200 (Agric. Biol. Chem., 48, 669, 1984), pLSA1 (Agric. Biol. Chem., 53, 277, 1989), pGEL1 (Proc. Natl. Acad. Sci., USA, 82, 4306 (1985), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol. 73, No. 20, p6378-6385], pPE167 (Appl. Environ. Microbiol. 2007, 73: 6378-6385), pPAC31 (WO 1998/12343), pUC19 (Gene, 33, 103, 1985), and pPA1 (JPS63-233798A).

The promoter in the case of using the above expression vector may be any promoter as long as it functions in cells of a microorganism belonging to the genus Escherichia, and examples thereof include a promoter derived from Escherichia coli, phage, or the like, such as a trp promoter, a gapA promoter, a lac promoter, a PL promoter, a PR promoter, or a PSE promoter. Examples thereof include a promoter which is artificially modified in design, such as a promoter with two trp promoters in series, a tac promoter, a trc promoter, a lacT5 promoter, a lacT7 promoter, or a letI promoter.

When a coryneform bacterium is used as a host cell into which the recombinant DNA of the present invention is to be introduced, examples of the expression vector include pCG1 (JPS57-134500A), pCG2 (JPS58-35197A), pCG4 (JPS57-183799A), pCG11 (JPS57-134500A), pCG 116, pCE54, and pCB101 (all JPS58-105999A), pCE51, pCE52, and pCE53 (all Molecular and General Genetics, 196, 175, 1984).

When the above expression vector is used, the promoter may be any promoter as long as it functions in cells of the coryneform bacterium, and examples thereof include a P54-6 promoter (Appl. Microbiol. Biotechnol., 53, p674-679, 2000).

When a yeast strain is used as a host cell into which the recombinant DNA of the present invention is to be introduced, examples of the expression vector include YEp13 (ATCC 37115), YEp24 (ATCC 37051), YCp50 (ATCC 37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP1 promoter.

The recombinant DNA of the present invention can be prepared by, for example, subjecting the DNA fragment prepared by the method of the above 2 to a restriction enzyme treatment or the like and inserting the DNA fragment into downstream of the promoter of the appropriate expression vector.

Here, by substituting a nucleotide such that the nucleotide sequence constituting the DNA of the present invention is an optimal codon for expression in a host cell, the expression level of the protein encoded by the DNA can be improved. Information on the frequency of codon use in a host cell can be obtained through a public database.

### 4. Transformant of Present Invention

A transformant of the present invention is a transformant obtained by transforming a host cell with the recombinant DNA comprising the DNA of the present invention according to the above 2. In the present description, the term "host cell" refers to an original cell to be transformed by gene transfer.

Specific examples of the transformant of the present invention include a microorganism with an enhanced activity of the protein of any one of [1] to [3] and enhanced fucose-containing carbohydrate productivity. Examples of the microorganism with an enhanced activity of the above protein of any one of above [1] to [3] include a microorganism with enhanced fucose-containing carbohydrate productivity as compared with a parent strain, which is obtained by transforming the parent strain with the recombinant DNA comprising the DNA of any one of [A1] to [A3].

Examples of the microorganism with enhanced fucose-containing carbohydrate productivity as compared with a parent strain, which is obtained by transforming the parent strain with the recombinant DNA include the following microorganisms i) to iii).
i) A microorganism in which a transcription amount of the DNA or a production amount of the protein encoded by the DNA is increased by introducing the recombinant DNA comprising the above DNA according to any one of [A1] to [A3] as an autonomously replicable plasmid into a parent strain or by incorporating the recombinant DNA into a chromosome of a parent strain.
ii) A microorganism in which fucose-containing carbohydrate productivity is enhanced by producing a protein having a transporting activity for a fucose-containing carbohydrate with an enhanced specific activity as the mutant protein of the above [2].
iii) A microorganism in which fucose-containing carbohydrate productivity is enhanced by producing a protein having a transporting activity for a fucose-containing carbohydrate with an enhanced specific activity as the homologous protein of the above [3].

As a method for confirming that the transcription amount of the above DNA according to any one of [A1] to [A3] or the production amount of the protein encoded by the DNA is increased, for example, it can be confirmed by measuring the transcription amount of the DNA by Northern blotting or the production amount of the protein by Western blotting, and comparing the transcription amount of the DNA or the production amount of the protein with that of a parent strain.

As a method for confirming that the specific activity of the protein having a transporting activity for a fucose-containing carbohydrate is enhanced, for example, it can be confirmed by obtaining a transformed strain by transforming a parent strain with a DNA encoding a mutant protein, culturing the transformant in a culture medium, measuring a specific activity from a generation amount of a fucose-containing carbohydrate accumulated in a culture product and an amount of the protein, and comparing the measured specific activity with a specific activity of a protein having a transporting activity for a fucose-containing carbohydrate and having no mutation introduced, which is measured in the same manner.

Examples of such a transformant of the present invention include an FucT/pSTV_YdeA strain and an FucT/pMW 1 18_MdfA strain to be described later in Examples.

The host cell into which the recombinant DNA of the present invention is to be introduced may be any of a prokaryote, a yeast, an animal cell, an insect cell, a plant cell, and the like. The host cell is preferably a prokaryote or a yeast strain, is more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and is most preferably a prokaryote such as Escherichia coli [e.g., Escherichia coli BL21 codon plus, Escherichia coli XL1-Blue, and Escherichia coli XL2-Blue (all manufactured by Agilent Technologies, Inc.), Escherichia coli BL21 (DE3) pLysS (manufactured by Merck Millipore Inc.), Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli W, Escherichia coli JM101, Escherichia coli W3110, Escherichia coli MG1655, Escherichia coli DH1, Escherichia coli MC1000, Escherichia coli W1485, Escherichia coli MP347, Escherichia coli NM522, Escherichia coli ATCC 9637, Serratia ficaria, Serratia fonticola, Serratia liquefaciens, Serratia marcescens, Bacillus subtilis, Bacillus amyloliquefaciens, Brevibacterium immariophilum [for example, Brevibacterium immariophilum ATCC 14068], Brevibacterium saccharolyticum [e.g., Brevibacterium saccharolyticum ATCC 14066], Corynebacterium ammoniagenes, Corynebacterium glutamicum [Corynebacterium glutamicum ATCC 13032, Corynebacterium glutamicum ATCC 14067, Corynebacterium glutamicum ATCC 13869], Corynebacterium acetoacidophilum [Corynebacterium acetoa cidophilum ATCC 13870], Microbacterium ammoniaphilum [Microbacterium ammoniaphilum ATCC 15354], or Pseudomonas (for example, Pseudomonas sp. D-0110), or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

Examples of the host cell include a microorganism having an ability to produce a fucose-containing carbohydrate, and a microorganism having a higher activity of a protein associated with biosynthesis of a fucose-containing carbohydrate than a parent strain is preferred. Examples of the protein associated with biosynthesis of a fucose-containing carbohydrate include GDP-fucose which is a reaction substrate for a fucosyltransferase, a receptor carbohydrate which is a reaction substrate for a fucosyltransferase, and a fucosyltransferase.

Examples of the host cell include the following 1) to 3).
1) A microorganism used as a host cell that is artificially endowed or enhanced with an ability to produce GDP-fucose, which is a reaction substrate for a fucosyltransferase
2) A microorganism used as a host cell that is artificially endowed or enhanced with an ability to supply a receptor carbohydrate, which is a reaction substrate for a fucosyltransferase
3) A microorganism used as a host cell that is artificially endowed or enhanced with a fucosyltransferase activity

Hereinafter, each host cell will be described.

### 1) A microorganism used as a host cell that is artificially endowed or enhanced with an ability to produce GDP-fucose, which is a reaction substrate for a fucosyltransferase

Specific examples of a method for endowing or enhancing the ability to produce GDP-fucose to a microorganism used as a host cell include a known method such as a method using various genetic manipulations (Metabolic Engineering (2017) 41: 23-38).

Examples of the ability to produce GDP-fucose include an ability to produce GDP-fucose from a saccharide. Examples of the method for artificially endowing or enhancing the ability to produce GDP-fucose from a saccharide to a microorganism used as a host cell include the following methods (1a) to (1d). These methods may be used alone or in combination.
(1a) A method of alleviating or releasing at least one mechanism for controlling a biosynthetic pathway for producing GDP-fucose from a saccharide
(1b) A method of enhancing expression of at least one enzyme associated with a biosynthetic pathway for producing GDP-fucose from a saccharide
(1c) A method of increasing the number of copies of at least one gene encoding an enzyme associated with a biosynthetic pathway for producing GDP-fucose from a saccharide
(1d) A method of weakening or blocking at least one metabolic pathway branching off from a biosynthetic pathway for producing GDP-fucose from a saccharide to a metabolic product other than a target substance

Specific examples of the mechanism for controlling a biosynthetic pathway for producing GDP-fucose from a saccharide include known mechanisms such as a control mechanism based on a transcription control factor (e.g., RcsA) associated with control of the biosynthetic pathway.

Specific examples of the enzyme associated with the biosynthetic pathway for producing GDP-fucose from a saccharide include known enzymes such as a mannose-6-phosphate isomerase, a phosphomannomutase, a mannose-1-phosphate guanylyltransferase, a GDP mannose-4,6-dehydratase, and a GDP-L-fucose synthase.

Specific examples of the metabolic pathway branching off from a biosynthetic pathway for producing GDP-fucose from a saccharide to a metabolic product other than a target substance include a known metabolic pathway such as a metabolic pathway from GDP-fucose to colanic acid.

### 2) A microorganism used as a host cell that is artificially endowed or enhanced with an ability to supply a receptor carbohydrate, which is a reaction substrate for a fucosyltransferase

Examples of the method for artificially endowing or enhancing an ability to supply a receptor carbohydrate to a microorganism used as a host cell include the following (2a) to (2g). These methods may be used alone or in combination.
(2a) A method of alleviating or releasing at least one mechanism for controlling a biosynthetic pathway for producing a receptor carbohydrate from a saccharide
(2b) A method of enhancing expression of at least one enzyme associated with a biosynthetic pathway for producing a receptor carbohydrate from a saccharide
(2c) A method of increasing the number of copies of at least one gene encoding an enzyme associated with a biosynthetic pathway for producing a receptor carbohydrate from a saccharide
(2d) A method of alleviating or releasing at least one mechanism for decomposing a receptor carbohydrate
(2e) A method of enhancing an expression of at least one enzyme associated with intracellular uptake of a receptor carbohydrate
(2f) A method of increasing the number of copies of at least one gene encoding an enzyme associated with intracellular uptake of a receptor carbohydrate
(2g) A method of weakening or blocking at least one metabolic pathway branching off from a receptor carbohydrate to a metabolic product other than a target substance

Examples of the receptor carbohydrate include N-acetylglucosamine, N-acetyllactosamine, galactose, fucose, sialic acid, glucose, or lactose, a combination thereof, and further a glycan containing these as a partial structure. Among them, lactose is preferred.

Specific examples of the enzyme associated with a biosynthetic pathway for producing a receptor carbohydrate from a saccharide include known enzymes such as an enzyme having a lactose synthase activity for producing lactose using glucose and UDP-galactose as substrates. Specific examples of the mechanism for decomposing a receptor carbohydrate include known enzymes such as β-galactosidase that catalyzes hydrolysis of lactose to produce glucose and galactose.

Specific examples of the enzyme associated with intracellular uptake of a receptor carbohydrate include known enzymes such as lactose permease associated with intracellular uptake of lactose. Specific examples of the method for endowing or enhancing an ability to supply a receptor carbohydrate include known methods such as a method of reducing or inactivating an activity of β-galactosidase by a genetic manipulation (Metabolic Engineering, 2017, 41: 23-38).

### 3) A microorganism used as a host cell that is artificially endowed or enhanced with a fucosyltransferase activity

Examples of the method for artificially endowing or enhancing a fucosyltransferase activity to a microorganism used as a host cell include the following (3a) and (3b). These methods may be used alone or in combination.
(3a) A method of enhancing expression of at least one fucosyltransferase
(3b) A method of increasing the number of copies of at least one gene encoding a fucosyltransferase

Examples of the fucosyltransferase include α1,2-fucosyltransferase, α1,3-fucosyltransferase, α1,4-fucosyltransferase, and α1,6-fucosyltransferase. Among these, α1,3-fucosyltransferase is preferred.

Specific examples of the α1,3-fucosyltransferase include the following proteins I) to III).
I) A protein consisting of the amino acid sequence represented by SEQ ID NO: 6
II) A mutant protein having an α1,3-fucosyltransferase activity and consisting of an amino acid sequence in which 1 to 20 amino acids are deleted, substituted, inserted, or added in the amino acid sequence represented by SEQ ID NO: 6
III) A homologous protein having an α1,3-fucosyltransferase activity and consisting of an amino acid sequence having an identity of 90% or more with the amino acid sequence represented by SEQ ID NO: 6

Examples of the method for introducing the recombinant DNA of the above 3 as an autonomously replicatable plasmid in a host cell include the above-described method using calcium ions, protoplast method, and electroporation method, a spheroplast method (Proc. Natl. Acad. Sci., USA, 81, 4889, 1984), and a lithium acetate method (J. Bacteriol., 153, 163, 1983).

Examples of the method for incorporating a recombinant DNA into a chromosome of a host cell include a homologous recombination method. Examples of the homologous recombination method include a method using a plasmid for homologous recombination that can be prepared by ligating with a plasmid DNA comprising a drug resistance gene that cannot autonomously replicate in a host cell to be introduced. Examples of the method using homologous recombination frequently used in Escherichia coli include a method of introducing a recombinant DNA using a homologous recombination system of a lambda phage (Proc. Natl. Acad. Sci. USA, 97, 6641 to 6645, 2000).

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the transformant obtained by the above method contains the DNA of the present invention according to the above 2 can be confirmed by, for example, in the case where the transformant is a transformant obtained by transforming a host cell having an ability to produce GDP-fucose or a receptor carbohydrate from a saccharide, culturing the transformant in a culture medium, and comparing the production amount of fucose-containing carbohydrate accumulated in the culture product with that of a parent strain.

### 5. Method for Producing Fucose-containing Carbohydrate of Present Invention

Examples of the method for producing a fucose-containing carbohydrate of the present invention include a method of producing a fucose-containing carbohydrate by a fermentation method including culturing the transformant of the above 4 in a culture medium to produce a fucose-containing carbohydrate in a culture product.

The transformant of the present invention used in the method of producing a fucose-containing carbohydrate by a fermentation method is preferably a transformant having an ability to produce GDP-fucose from a saccharide.

As the transformant of the present invention used in the method of producing a fucose-containing carbohydrate by a fermentation method, a transformant having an ability to produce a receptor carbohydrate may be used.

The method of culturing the transformant in the above 4 can be performed according to a method generally used for culturing a microorganism.

As a culture medium for culturing the transformant, any of a natural culture medium and a synthetic culture medium may be used as long as the culture medium contains a carbon source, a nitrogen source, an inorganic salt, and the like that can be assimilated by the transformant and can efficiently culture the transformant.

Any carbon source may be used as long as it can be assimilated by the transformant, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolysate, a soybean meal, a soybean meal hydrolysate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate. A receptor carbohydrate such as lactose as a precursor of a fucose-containing carbohydrate may be added to the culture medium, or GTP, mannose, or the like as a precursor of GDP-fucose may be added to the culture medium.

In the method of producing a fucose-containing carbohydrate by a fermentation method, when the transformant to be used does not have the ability to produce GDP-fucose, GDP-fucose is added to the culture medium during culture. In the method of producing a fucose-containing carbohydrate by a fermentation method, when the transformant to be used does not have the ability to produce GDP-fucose, instead of adding GDP-fucose to the culture medium, GDP-fucose may be supplied to the transformant of the present invention by culturing microorganisms having the ability to produce GDP-fucose from a saccharide simultaneously with the transformant of the present invention.

In order to supply or enhance GTP, which is a precursor of GDP-fucose, microorganisms having the ability to produce GTP may be simultaneously cultured. Examples of the microorganism having the ability to produce GTP include known microorganisms such as a microorganism in which expression of an enzyme associated with a biosynthetic pathway of GTP is enhanced by various genetic manipulations (Biotechnol Bioeng, Sep3: 2019, 2412 to 2417).

In the method of producing a fucose-containing carbohydrate by a fermentation method, when the transformant to be used does not have the ability to produce a receptor carbohydrate such as lactose, a receptor carbohydrate such as lactose is added to the culture medium during culture.

In the method of producing a fucose-containing carbohydrate by a fermentation method, when the transformant to be used does not have the ability to produce a receptor carbohydrate such as lactose, instead of adding a receptor carbohydrate such as lactose to the culture medium during culture, a receptor carbohydrate such as lactose may be supplied to the transformant of the present invention by culturing microorganisms having the ability to produce a receptor carbohydrate such as lactose from a saccharide simultaneously with the transformant of the present invention.

The culture is generally preferably performed under preferred conditions such as shaking culture or deep aeration stirring culture. The culture temperature is generally 15°C to 40°C, and the culture time is generally 5 hours to 7 days. The pH of the culture solution during culture is generally maintained at 3.0 to 9.0. The pH is adjusted using an inorganic or organic acid, an alkaline solution, urea, calcium carbonate, ammonia, or the like.

### [Analysis Example]

In Examples, analysis and quantification of 3-fucosyllactose were performed by the following procedure. A culture solution containing microorganisms after culture was centrifuged, and the supernatant was collected. 3-Fucosyllactose contained in the supernatant was analyzed by a carbohydrate analyzer ICS-5000 (manufactured by Thermo Fisher Scientific K.K.).

### [Analysis Conditions]

Column: CarboPAC PA1
Column temperature: 25°C
Mobile phase: (mobile phase A) water
(mobile phase B) 500 mmol/L sodium hydroxide
(mobile phase C) 300 mmol/L sodium acetate
Mobile phase A, mobile phase B, and mobile phase C mixing ratio:
   (0 minutes to 10 minutes) 80:20:0
   (10 minutes to 15 minutes) gradient from 80:20:0 to 70:20:10
   (15 minutes to 17 minutes) gradient from 70:20:10 to 0:20:80
   (17 minutes to 25 minutes) 0:20:80
   (25 minutes to 35 minutes) 80:20:0
Flow rate: 1.0 mL/min
Detector: pulsed amperometric detector

### Example

Examples of the invention are shown below, but the invention is not limited to these Examples.

### [Example 1] Construction of microorganism used for production of 3-fucosyllactose

### (1) Acquisition of DNA fragment to be used as marker for gene deletion

PCR was performed using, as a primer set, DNAs consisting of the nucleotide sequences shown in "Primer set" in Table 1 and using, as a template, a DNA described in "Template" in Table 1 to obtain each amplified DNA fragment.

**[Table 1]**

| Primer set (SEQ ID NO:) | Template | Amplified DNA fragment |
|---|---|---|
| 8 and 9 | pHSG396 (manufactured by Takara Bio Inc.) | cat |
| 10 and 11 | Genomic DNA of Bacillus subtilis 168 strain | sacB |

A genomic DNA of a Bacillus subtilis 168 strain was prepared by a standard method. The cat of the amplified DNA fragment contains about 200 bp upstream to about 50 bp downstream of the cat gene on pHSG 396. The sacB of the amplified DNA fragment contains about 300 bp upstream to about 100 bp downstream of the sacB gene on the genomic DNA of the Bacillus subtilis 168 strain.

Next, PCR was performed using, as a template, the cat and sacB of the amplified DNA fragment and using, as a primer set, DNAs consisting of the nucleotide sequences represented by SEQ ID NOs: 8 and 11 to obtain a DNA fragment comprising the cat gene and the sacB gene (hereinafter, referred to as cat-sacB).

### (2) Construction of Escherichia coli with loss of β-galactosidase activity, lactose permease activity, and colanic acid producing activity

Escherichia coli deficient in DNA encoding β-galactosidase (hereinafter, referred to as lacZ gene), DNA encoding lactose permease (hereinafter, referred to as lacY gene), and DNA encoding a colanic acid production-related protein (hereinafter, referred to as wcaJ, wzxC, wcaK, wcaL, or wcaM gene) was constructed by the following method. Note that the lacZ and lacY (hereinafter, referred to as lacZY), and wcaJ, wzxC, wcaK, wcaL, and wcaM (hereinafter, referred to as wcaJ-wzxC-wcaKLM) each form an operon on the Escherichia coli genome.

PCR was performed using, as a template, a genomic DNA of the Escherichia coli W3110 strain prepared by an ordinary method and using, as a primer set, DNAs consisting of the nucleotide sequences shown in "Primer set" in Table 2 to amplify each DNA fragment.

**[Table 2]**

| Primer set (SEQ ID NO:) | Amplified DNA fragment | Remark |
|---|---|---|
| 12 and 13 | lacZ upstream 1 | Sequences at 5' ends of nucleotide sequences represented by SEQ ID NOs: 8 and 12 are complementary |
| 14 and 15 | lacY downstream 1 | Sequences at 5' ends of nucleotide sequences represented by SEQ ID NOs: 11 and 14 are complementary |
| 13 and 16 | lacZ upstream 2 | Sequences at 5' ends of nucleotide sequences represented by SEQ ID NOs: 16 and 17 are complementary |
| 15 and 17 | lacY downstream 2 | |

The lacZ upstream 1 and lacZ upstream 2 include a region from an initiation codon of the lacZ gene to about 1000 bp upstream of the initiation codon. The lacY downstream 1 and a lacY downstream 2 include about 50 bp to about 1000 bp downstream of a stop codon of the lacY gene.

PCR was performed using, as a template, a mixture of the lacZ upstream 1, the lacY downstream 1, and the cat-sacB fragment at an equimolar ratio and using, as a primer set, DNAs consisting of the nucleotide sequences represented by SEQ ID NOs: 13 and 15 to obtain a DNA fragment consisting of a sequence with the cat-sacB fragment inserted in a region around the lacZ and lacY genes (hereinafter, referred to as lacZY::cat-sacB).

PCR was performed using, as a template, a mixture of the lacZ upstream 2 and the lacY downstream 2 at an equimolar ratio and using, as a primer set, DNAs consisting of the nucleotide sequences represented by SEQ ID NOs: 13 and 15 to obtain a DNA fragment consisting of a sequence with the lacZ upstream and the lacY downstream directly linked to each other without lacZ and lacY (hereinafter, referred to as ΔlacZY).

The lacZY::cat-sacB fragment was introduced into a W3110 strain carrying a plasmid pKD46 comprising a gene encoding λ recombinase [Datsenko, K. A., Wainer, B. L. L., Proc. Natl. Acad.Sci, USA, Vol. 97, 6640-6645, (2000)] by an electroporation method to obtain a transformant (a transformant with the lacZ and lacY genes substituted with lacZY::cat-sacB) exhibiting chloramphenicol resistance and sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant without pKD46) exhibiting ampicillin sensitivity was further obtained. The transformant was named W3110ΔlacZY.

Similarly, PCR was performed using, as a template, the genomic DNA of the W3110 strain and using, as a primer set, DNAs consisting of the nucleotide sequences shown in "Primer set" in Table 3 to obtain each amplified DNA fragment.

**[Table 3]**

| Primer set (SEQ ID NO:) | Amplified DNA fragment | Remark |
|---|---|---|
| 18 and 19 | wcaJ upstream 1 | Sequences at 5' ends of nucleotide sequences represented by SEQ ID NOs: 8 and 18 are complementary |
| 20 and 21 | wcaM downstream 1 | Sequences at 5' ends of nucleotide sequences represented by SEQ ID NOs: 11 and 20 are complementary |
| 19 and 22 | wcaJ upstream 2 | Sequences at 5' ends of nucleotide sequences represented by SEQ ID NOs: 22 and 23 are complementary |
| 21 and 23 | wcaM downstream 2 | |

The wcaJ upstream 1 and the wcaJ upstream 2 include a region from an initiation codon of the wcaJ gene to about 1000 bp upstream of the initiation codon. The wcaM downstream 1 and the wcaM downstream 2 include a region from a stop codon of the wcaM gene to about 1000 bp downstream of the stop codon.

PCR was performed using, as a template, a mixture of the wcaJ upstream 1, the wcaM downstream 1, and the cat-sacB fragment at an equimolar ratio and using, as a primer set, DNAs consisting of the nucleotide sequences represented by SEQ ID NOs: 19 and 21 to obtain a DNA fragment consisting of a sequence with the cat-sacB fragment inserted in a region around a wcaJ-wzxC-wcaKLM operon (hereinafter, referred to as wcaJ-wzxC-wcaKLM:: cat-sacB).

PCR was performed using, as a template, a mixture of the wcaJ upstream 2 and the wcaM downstream 2 at an equimolar ratio and using, as a primer set, DNAs consisting of the nucleotide sequences represented by SEQ ID NOs: 19 and 21 to obtain a DNA fragment consisting of a sequence with the wcaJ upstream and the wcaM downstream directly linked to each other without wcaJ-wzxC-wcaKLM (hereinafter, referred to as ΔwcaJ-wzxC-wcaKLM).

The wcaJ-wzxC-wcaKLM::cat-sacB fragment was introduced into the W3110 ΔlacZY strain constructed as described above by the electroporation method to obtain a transformant (a transformant with wcaJ-wzxC-wcaKLM substituted with wcaJ-wzxC-wcaKLM::cat-sacB) exhibiting chloramphenicol resistant and sucrose sensitivity.

The ΔwcaJM fragment was introduced into the transformant by the electroporation method to obtain a transformant (a transformant with wcaJ-wzxC-wcaKLM::cat-sacB substituted with ΔwcaJ-wzxC-wcaKLM) exhibiting chloramphenicol sensitivity and sucrose resistance. Further, a transformant (a transformant without pKD46) exhibiting ampicillin sensitivity was obtained. The transformant was named T166.

### (3) Construction of microorganism having α1,3-fucosyltransferase activity

PCR was performed using, as a primer set, DNAs consisting of the nucleotide sequences shown "Primer set" in Table 4 and using, as a template, a DNA described in "Template" in Table 4 to obtain each amplified DNA fragment.

**[Table 4]**

| Primer set (SEQ ID NO:) | Template | Amplified DNA fragment |
|---|---|---|
| 24 and 25 | Genomic DNA of Escherichia coli W3110 strain | rcsA |
| 26 and 28 | Genomic DNA of Bacteroides reticulotermitis JCM10512 strain | cBrFucT upstream |
| 29 and 30 | DNA consisting of nucleotide sequence represented by SEQ ID NO: 7 | cBrFucT midstream |
| 27 and 31 | Genomic DNA of Bacteroides reticulotermitis JCM10512 strain | cBrFucT downstream |
| 32 and 33 | Genomic DNA of Escherichia coli W3110 strain | lacY |

Genomic DNAs of the Escherichia coli W3110 strain and the Bacteroides reticulotermitis JCM 10512 strain were prepared by an ordinary method. A DNA consisting of the nucleotide sequence represented by SEQ ID NO: 7 is a DNA obtained by codon-optimizing, for expression in Escherichia coli, a partial sequence of a gene encoding Bacteroides fragilis ATCC25285-derived α1,3-fucosyltransferase. The nucleotide sequences represented by SEQ ID NOs: 25 and 26, SEQ ID NOs: 28 and 29, SEQ ID NOs: 30 and 31, and SEQ ID NOs: 27 and 32 comprise complementary sequences at each 5' end.

First, PCR was performed using, as a template, a mixture of three fragments of the cBrFucT upstream, the cBrFucT midstream, and the cBrFucT downstream at an equimolar ratio and using, as a primer set, DNAs consisting of the nucleotide sequences represented by SEQ ID NOs: 26 and 27 to obtain a DNA fragment with the three fragments linked (hereinafter, referred to as a cBrFucT fragment).

PCR was performed using, as a template, a mixture of rcsA, cBrFucT, and the lacY fragment at an equimolar ratio and using, as a primer set, DNAs consisting of the nucleotide sequences represented by SEQ ID NOs: 34 and 35 to obtain a DNA fragment with the three fragments linked (hereinafter, referred to as rcsA-cBrFucT-lacY).

PCR was performed using, as a primer set, DNAs consisting of the nucleotide sequences represented by SEQ ID NOs: 36 and 37 and using, as a template, a plasmid pPE 167 (Appl. Environ. Microbiol. 2007, 73: 6378-6385) to obtain a vector fragment of about 4.4 kb. In this case, the nucleotide sequences represented by SEQ ID NOs: 34 and 37 and SEQ ID NOs: 35 and 36 comprise complementary sequences at each 5' end.

The rcsA-cBrFucT-lacY fragment and the vector fragment obtained above were ligated using an In-Fusion HD Cloning Kit (manufactured by Takara Bio Inc.) to obtain an α1,3-fucosyltransferase expression plasmid.

By transforming theT166 strain constructed in the above (2) using the above α1,3-fucosyltransferase expression plasmid, a microorganism expressing α1,3-fucosyltransferase was constructed and named FucT strain.

### (4) Construction of microorganism with enhanced expression of Escherichia coli-derived transporter gene

An Escherichia coli-derived transporter gene (ydeA) belonging to drug:H+ antiporter-1 family was placed under the uspA promoter, and an Escherichia coli strain comprising the gene expression plasmid was constructed by the following method.

PCR was performed using, as a template, an expression vector pSTV29 (Takara Bio Inc.) and using, as a primer set, oligonucleotides consisting of the nucleotide sequences represented by SEQ ID NOs: 38 and 39 to obtain a pSTV29 vector fragment.

PCR was performed using, as a template, a chromosomal DNA of an Escherichia coli ATCC9637 strain and using, as a primer set, oligonucleotides consisting of the nucleotide sequences represented by SEQ ID NOs: 40 and 41 to amplify a DNA fragment comprising the uspA promoter. In this case, the nucleotide sequences represented by SEQ ID NOs: 38 and 41 and SEQ ID NOs: 39 and 40 comprise complementary sequences at each 5' end.

The obtained DNA fragment comprising the uspA promoter was ligated to the pSTV29 vector fragment using an In-Fusion HD Cloning Kit (manufactured by Takara Bio Inc.) to obtain an expression vector pSTV.

PCR was performed using, as a template, the chromosomal DNA of Escherichia coli W3110 strain and using, as a primer set, oligonucleotides consisting of the nucleotide sequences represented by SEQ ID NOs: 43 and 44 to amplify a DNA fragment encoding the YdeA protein (a protein comprising the amino acid sequence represented by SEQ ID NO: 2). In this case, the nucleotide sequences represented by SEQ ID NOs: 41 and 43 and SEQ ID NOs: 42 and 44 comprise complementary sequences at each 5' end.

The obtained DNA fragment encoding YdeA was ligated to the pSTV vector fragment using an In-Fusion HD Cloning Kit (manufactured by Takara Bio Inc.) to obtain an expression vector pSTV_YdeA.

Subsequently, an Escherichia coli-derived transporter gene (mdfA) belonging to drug:H+ antiporter-1 family was placed under a lac promoter, and an Escherichia coli strain having the gene expression plasmid was constructed by the following method.

PCR was performed using, as a template, an expression vector pMW 118 (manufactured by Nippon Gene Co., Ltd.) and using, as a primer set, oligonucleotides consisting of the nucleotide sequences represented by SEQ ID NOs: 45 and 46 to obtain a pMW118 vector fragment.

PCR was performed using, as a template, the chromosomal DNA of Escherichia coli W3110 strain and using, as a primer set, oligonucleotides consisting of the nucleotide sequences represented by SEQ ID NOs: 47 and 48 to amplify a DNA fragment encoding the MdfA protein (a protein comprising the amino acid sequence represented by SEQ ID NO: 4). In this case, the nucleotide sequences represented by SEQ ID NOs: 45 and 48 and SEQ ID NOs: 46 and 47 comprise complementary sequences at each 5' end.

The obtained DNA fragment encoding MdfA was ligated to the pMW118 vector fragment using an In-Fusion HD Cloning Kit (manufactured by Takara Bio Inc.) to obtain an expression vector pMW118_MdfA.

The expression vectors pSTV and pMW 118 and the expression plasmids pSTV_YdeA and pMW118_MdfA obtained above were used to transform the FucT strains constructed in Example 1(3) to obtain an FucT/pSTV strain, an FucT/pMW118 strain, an FucT/pSTV_YdeA strain, and an FucT/pMW 118_MdfA strain.

### [Example 2] Production of 3-fucosyllactose by fermentation method using microorganism strongly expressing transporter

The FucT/pSTV strain, FucT/pMW118 strain, FucT/pSTV_YdeA strain, and FucT/pMW 118_MdfA strain obtained in Example 1 were cultured on LB plates at 30°C for 24 hours, inoculated into large-sized test tubes each containing 4 mL of LB culture medium containing 100 mg/L of kanamycin and 25 mg/L of chloramphenicol (pSTV29-derived plasmid expression strain) or 100 mg/L of ampicillin (pMW118-derived plasmid expression strain), and shaking-cultured at 30°C for 18 hours.

Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (adjusted to pH 7.2 by aqueous sodium hydroxide, except for glucose, lactose monohydrate, and magnesium sulfate heptahydrate, and then autoclaved) (aqueous solutions containing glucose, lactose monohydrate, and magnesium sulfate heptahydrate were separately prepared, autoclaved, cooled, and then mixed)] containing 100 mg/L of kanamycin and 25 mg/L of chloramphenicol (pSTV29-derived plasmid expression strain) or 100 mg/L of ampicillin (pMW118-derived plasmid expression strain), and shaking-cultured at 30°C for 28 hours. The FucT/pMW 118 strain and the FucT/pMW 118_MdfA strain were added with IPTG at 1 mM after 5 hours from the start of culture.

After completion of the culture, the culture solution was appropriately diluted and centrifuged, and 3-fucosyllactose contained in the supernatant was analyzed by a carbohydrate analyzer ICS-5000. The results are shown in Table 5.

**[Table 5]**

| Strain name | Expression gene | 3-fucosyllactose (g/L) |
|---|---|---|
| FucT/pSTV | - | 0.24 |
| FucT/pSTV_YdeA | ydeA | 5.71 |
| FucT/pMW118 | - | 0.26 |
| FucT/pMW118_MdfA | mdfA | 5.84 |

As shown in Table 5, compared to the FucT/pSTV strain and the FucT/pMW 118 strain, the FucT/pSTV_YdeA strain and the FucT/pMW 118_MdfA strain exhibited remarkably high 3-fucosyllactose productivity.

Accordingly, it is found that the 3-fucosyllactose productivity is improved by strongly expressing YdeA or MdfA belonging to the drug:H+ antiporter-1 family.

Although the present invention has been described in detail with reference to specific embodiments, it will be apparent to those skilled in the art that various changes and modifications can be made without departing from the spirit and scope of the invention. The present application is based on a Japanese Patent Application No. 2021-018484 filed on February 8, 2021, the entire contents of which are incorporated herein by reference. All references cited herein are incorporated in their entirety.

### Sequence Listing Free Text

SEQ ID NO: 1: nucleotide sequence of ydeA derived from E. coli W3110
SEQ ID NO: 2: amino acid sequence of YdeA derived from E. coli W3110
SEQ ID NO: 3: nucleotide sequence of mdfA derived from E. coli W3110
SEQ ID NO: 4: amino acid sequence of MdfA derived from E. coli W3110
SEQ ID NO: 5: nucleotide sequence of chimeric FucT
SEQ ID NO: 6: amino acid sequence of chimeric FucT
SEQ ID NO: 7: partial sequence of codon-optimized BfFucT
SEQ ID NOs: 8 and 9: nucleotide sequences of cat amplification primers
SEQ ID NOs: 10 and 11: nucleotide sequences of sacB amplification primers
SEQ ID NOs: 12 and 13: nucleotide sequences of lacZ upstream 1 amplification primers
SEQ ID NOs: 14 and 15: nucleotide sequences of lacY downstream 1 amplification primers
SEQ ID NO: 16: nucleotide sequence of lacZ upstream 2 amplification primer
SEQ ID NO: 17: nucleotide sequence of lacY downstream 2 amplification primer
SEQ ID NOs: 18 and 19: nucleotide sequences of wcaJ upstream 1 amplification primers
SEQ ID NOs: 20 and 21: nucleotide sequences of wcaM downstream 1 amplification primers
SEQ ID NO: 22: nucleotide sequence of wcaJ upstream 2 amplification primer
SEQ ID NO: 23: nucleotide sequence of wcaM downstream 2 amplification primer
SEQ ID NOs: 24 and 25: nucleotide sequences of rcsA amplification primers
SEQ ID NOs: 26 and 28: nucleotide sequences of cBrFucT upstream amplification primers
SEQ ID NO: 27 and 31: nucleotide sequence of cBrFucT downstream amplification primers
SEQ ID NOs: 29 and 30: nucleotide sequences of cBrFucT midstream amplification primers
SEQ ID NOs: 32 and 33: nucleotide sequences of lacY amplification primers
SEQ ID NOs: 34 and 35: nucleotide sequences of rcsA-cBrFucT-lacY amplification primers
SEQ ID NOs: 36 and 37: nucleotide sequences of pPE167 amplification primers
SEQ ID NOs: 38 and 39: nucleotide sequences of pSTV29 amplification primers
SEQ ID NOs: 40 and 41: nucleotide sequences of PuspA amplification primers
SEQ ID NO: 42: nucleotide sequence of pSTV-PuspA amplification primer
SEQ ID NOs: 43 and 44: nucleotide sequences of ydeA amplification primers
SEQ ID NOs: 45 and 46: nucleotide sequences of pMW118 amplification primers
SEQ ID NOs: 47 and 48: nucleotide sequences of mdfA amplification primers

## Claims

1. A protein of any one of [1] to [3] below,
[1] a protein consisting of the amino acid sequence represented by SEQ ID NO: 2 or 4,
[2] a mutant protein having a transporting activity for a fucose-containing carbohydrate and consisting of an amino acid sequence in which 1 to 20 amino acids are deleted, substituted, inserted, or added in the amino acid sequence represented by SEQ ID NO: 2 or 4, and
[3] a homologous protein having a transporting activity for a fucose-containing carbohydrate and consisting of an amino acid sequence having an identity of 90% or more with the amino acid sequence represented by SEQ ID NO: 2 or 4.

2. The protein according to claim 1, wherein the fucose-containing carbohydrate is an oligosaccharide.

3. A DNA comprising the nucleotide sequence represented by SEQ ID NO: 1 or 3 or a homologous sequence thereof and encoding the protein according to claim 1 or 2.

4. A recombinant DNA comprising the DNA according to claim 3.

5. A transformant obtained by transforming a host cell with the recombinant DNA according to claim 4.

6. The transformant according to claim 5, which is a microorganism with an enhanced activity of the protein of any one of [1] to [3] according to claim 1 and enhanced productivity of the fucose-containing carbohydrate.

7. The transformant according to claim 6, wherein the microorganism is Escherichia coli having an ability to produce the fucose-containing carbohydrate.

8. A method for producing a fucose-containing carbohydrate, comprising: culturing the transformant according to any one of claims 5 to 7 in a culture medium to produce the fucose-containing carbohydrate in a culture product.

9. The production method according to claim 8, wherein the fucose-containing carbohydrate is an oligosaccharide.

10. The production method according to claim 9, wherein the oligosaccharide is 3-fucosyllactose.
